# EUROPÄISCHE PATENTANMELDUNG

(11) **EP 0 780 384 A2**
(43) Veröffentlichungstag der Anmeldung: **25.06.1997**
(21) Anmeldenummer: 96119776.1
(22) Anmeldetag: 10.12.1996
(51) Int. Cl.: C07D 277/32, C07D 277/36

(54) **Verfahren zur Herstellung von 2-Chlor-5-chlormethylthiazol**

(30) Priorität: 22.12.1995 DE 19548417
(71) Anmelder: BAYER AG, 51368 Leverkusen (DE)
(72) Erfinder: Kraatz, Udo, Dr., 51375 Leverkusen (DE)

(57) **Zusammenfassung**

Die Erfindung betrifft ein neues Verfahren zur Herstellung von 2-Chlor-5-chlormethylthiazol, durch Umsetzung von 5-Methylen-1,3-thiazolidin-2-thionen mit einem Chlorierungsmittel.

## Beschreibung

Die Erfindung betrifft ein neues Verfahren zur Herstellung von 2-Chlor-5-chlormethylthiazol.

Es ist bekannt, daß man 2-Chlor-5-chlormethylthiazol erhält, wenn man Allylisothiocyanat (Allylsenföl) der Formel (A) mit einem Chlorierungsmittel gemäß dem folgenden Reaktionsschema umsetzt: 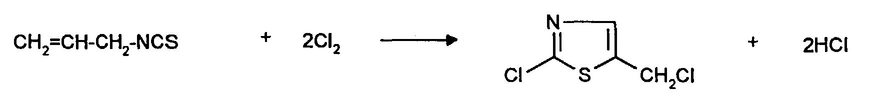 (vgl. hierzu EP-A 0260560).

Ferner ist bekannt daß man 2-Chlor-5-chlormethylthiazol erhalten kann, wenn man Allylisothiocyanate der Formel (B) mit einem Chlorierungsmittel gemäß dem folgenden Reaktionsschema umsetzt: 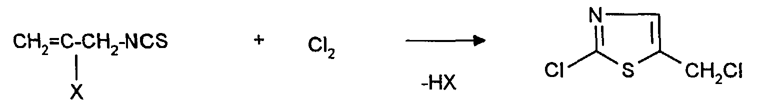 X = Abgangsgruppe
(vgl. hierzu EP-A 0446913).

Diese Verfahren haben jedoch den Nachteil, daß ein beträchtlicher Überschuß an Chlorierungsmittel verwendet wird, in großer Verdünnung gearbeitet werden muß und ein exaktes Einhalten der Reaktionstemperatur erforderlich ist.

Darüber hinaus muß die sich im Reaktionsverlauf bildende stabile Zwischenstufe in einem zusätzlichen Reaktionsschritt exotherm in das gewünschte Endprodukt überführt werden. Das erfordert, insbesondere bei der Durchführung im technischen Maßstab einen zusätzlichen Überwachungsaufwand.

Es wurde nun gefunden, daß man 2-Chlor-5-chlormethyl-thiazol der Formel (I) 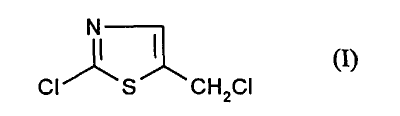 in guten Ausbeuten und hoher Reinheit erhält, wenn man 5-Methylen-1,3-thiazolidin-2-thione der Formel (II) 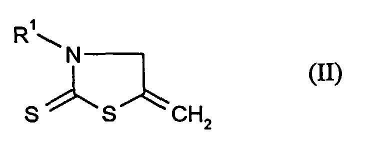 in welcher
- R¹: für Wasserstoff oder die Gruppierung R²-CO- steht, wobei
- R²: für Alkyl oder gegebenenfalls substituiertes Phenyl steht,
mit einem Chlorierungsmittel, gegebenenfalls in Gegenwart eines Verdünnungsmittels umsetzt.

Überraschenderweise kann nach dem erfindungsgemäßen Verfahren das 2-Chlor-5-chlormethyl-thiazol der Formel (I) in guten Ausbeuten und in hoher Reinheit erhalten werden, obwohl nach dem Stand der Technik erwartet werden mußte, daß die >N-CS-Gruppe unter solch milden Chlorierungsbedingungen nicht entschwefelt wird (vgl. z.B. Org. Synthesis 51, 139 (1971). Außerdem war nicht zu erwarten, daß die cyclische Struktur des Dithiocarbamats bei einer Chlorierung erhalten bleibt (vgl. z.B. Houben Weyl, Methoden der organischen Chemie, Bd. 5/3, S. 648 (1962).

Die erfindungsgemäße Umsetzung hat somit den Vorteil einer einfachen und schnellen Verfahrensdurchführung, wobei bereits ohne Reinigungsschritte hohe Rohausbeuten erzielt werden. Durch das Nichtauftreten einer stabilen Zwischenstufe entfällt auch der nachgeschaltete thermolytische Reaktionsschritt.

Verwendet man beispielsweise 5-Methylen-1,3-thiazolidin-2-thion als Ausgangsstoff und elementares Chlor als Chlorierungsmittel, so kann der Reaktionsablauf des erfindungsgemäßen Verfahrens durch das folgende Formelschema skizziert werden:

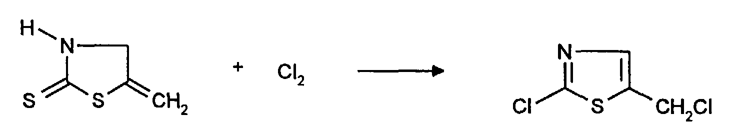

Die beim erfindungsgemäßen Verfahren als Ausgangsstoffe zu verwendenden 5-Methylen-1,3-thiazolidin-2-thione sind durch die Formel (II) allgemein definiert. In der Formel (II) steht R¹ vorzugsweise für Wasserstoff oder die Gruppierung R²-CO-, wobei R² vorzugsweise für geradkettiges oder verzweigtes C₁-C₄-Alkyl, wie insbesondere Methyl, Ethyl oder Isopropyl; sowie für gegebenenfalls einfach bis zweifach gleich oder verschieden substituiertes Phenyl steht, wobei als Substituenten Methyl, Ethyl, Fluor, Chlor, Nitro und Cyano genannt seien.

Die 5-Methylen-1,3-thiazolidin-2-thione der Formel (II) sind prinzipiell bekannt ( vgl. Liebigs Ann. Chem. 1985, 58ff).

Als Chlorierungsmittel kommen elementares Chlor sowie bei den Reaktionsbedingungen chlorabspaltende Verbindungen infrage, wie z.B. Sulfurylchlorid oder Phosgen.

Das erfindungsgemäße Verfahren wird vorzugsweise in Gegenwart eines Verdünnungsmittels durchgeführt.

Als Verdünnungsmittel kommen übliche organische Lösungsmittel infrage. Hierzu gehören vorzugsweise chlorierte aliphatische bzw. aromatische Kohlenwasserstoffe, wie Dichlormethan, Trichlormethan, Trichlorethylen, Tetrachlorethylen und Tetrachlorkohlenstoff bzw. Chlorbenzol oder Dichlorbenzol.

Die Reaktionstemperaturen können bei der Durchführung des erfindungsgemäßen Verfahrens in einem größeren Bereich variiert werden. Im allgemeinen arbeitet man bei Temperaturen zwischen -80°C und 150°C, vorzugsweise bei Temperaturen zwischen -30°C und 80°C.

Reaktionsdurchführung und Aufarbeitung erfolgen in allgemein üblicher Art und Weise (vgl. auch die Herstellungsbeispiele).

Wird beim erfindungsgemäßen Verfahren anstelle eines Chlorierungsmittels ein entsprechendes Bromierungsmittel eingesetzt, erhält man das 2-Brom-5-brommethylthiazol der Formel (III)

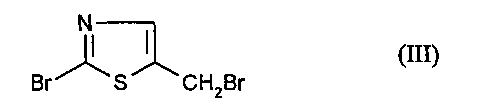

Das 2-Brom-5-brommethylthiazol der Formel (III) ist bekannt (vgl. EP-A 0376279).

Das nach dem erfindungsgemäßen Verfahren herzustellende 2-Chlor-5-chlormethylthiazol der Formel (I) kann als Zwischenprodukt zur Herstellung von biologisch aktiven Verbindungen, beispielsweise von Insektiziden verwendet werden (vgl. z.B. EP-A 0192060).

### Herstellungsbeispiele

### Beispiel 1

Zur Suspension von 20 g (0.15 Mol) 5-Methylen-1.3-thiazolidin-2-thion in 200 ml Chloroform leitet man bei -10°C einen Chlorgasstrom ein. Nach kurzer Zeit erhält man eine klare Lösung, die sich durch ausfallende Produkte trübt. Sobald kein Chlor mehr aufgenommen wird, rührt man noch 10 Minuten bei -10°C, läßt die Temperatur auf 20°C steigen und wäscht mehrmals mit Wasser. Anschließend wird das Lösungsmittel im Vakuum entfernt.

Man erhält 26.9 g gelbes, flüssiges Rohprodukt mit einer GC-Reinheit von 87 %, was einer theoretischen Ausbeute von 92.8 % entspricht.

Das Rohprodukt läßt sich durch Chromatographie aus Kieselgel im System Chloroform/Essigester (4:1) noch weiter aufreinigen.

Man erhält 10.6 g (GC-Reinheit >99 %) 2-Chlor-5-chlormethylthiazol vom Schmelzpunkt 32°C.

### Beispiel 2

In die Lösung von 2,6 g (0.015 Mol) 3-Acetyl-5-methylen-1.3-thiazolidin-2-thion in 50 ml Chloroform leitet man bei 0°C Chlorgas ein (Dauer 15 Minuten). Man läßt dann noch 20 Minuten bei 20°C weiterrühren, wäscht die Lösung zweimal mit Wasser und engt die organische Phase nach dem Trocknen über Magnesiumsulfat ein.

Man erhält eine Rohausbeute von 2,4 g als gelbes Öl, das nach der GC-Analytik 41 % an gewünschtem 2-Chlor-5-chlormethyl-1,3-thiazol enthält.

## Patentansprüche

1. Verfahren zur Herstellung von 2-Chlor-5-chlormethylthiazol der Formel (I) 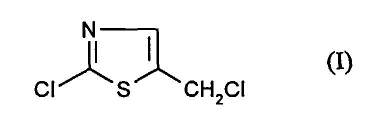 dadurch gekennzeichnet, daß man 5-Methylen-1,3-thiazolidin-2-thione der Formel (II) 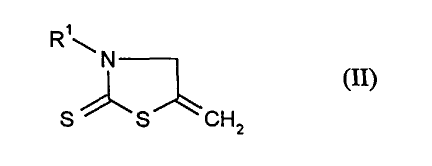 in welcher
R¹ für Wasserstoff oder die Gruppierung R²-CO- steht, wobei
R² für Alkyl oder gegebenenfalls substituiertes Phenyl steht,
mit einem Chlorierungsmittel, gegebenenfalls in Gegenwart eines Verdünnungsmittels umsetzt.
